(19) Europäisches Patentamt
European Patent Office
Office européen des brevets

(11) Publication number: **0 585 728 A1**

(12) **EUROPEAN PATENT APPLICATION**

(21) Application number: **93113176.7**

(51) Int. Cl.5: **A61K 31/385**

(22) Date of filing: **17.08.93**

(30) Priority: **29.08.92 JP 254130/92**

(43) Date of publication of application:
**09.03.94 Bulletin 94/10**

(84) Designated Contracting States:
**AT BE CH DE DK ES FR GB GR IT LI LU NL SE**

(71) Applicant: **NIHON NOHYAKU CO., LTD.**
**1-2-5, Nihonbashi**
**Chuo-ku Tokyo(JP)**

(72) Inventor: **Takeichi, Noritoshi**
**1-38, Minamijurokujonishi-13-chome**
**Chuo-ku, Sapporo-shi(JP)**
Inventor: **Nagayasu, Hiroki**
**Kento Hausu 2, 4-3, Asabucho-3-chome**
**Kita-ku, Sapporo-shi(JP)**
Inventor: **Hamada, Junichi**
**Rapoto 22-101**
**11-23,Shinkawa 2jo-2-chome**
**Kita-ku**
**Sapporo-Shi Hokkaido(JP)**
Inventor: **Konaka, Shigeo**
**3-13, Tagasugi-2-chome**
**Sanda-shi(JP)**

(74) Representative: **Patentanwälte Grünecker,**
**Kinkeldey, Stockmair & Partner**
**Maximilianstrasse 58**
**D-80538 München (DE)**

(54) **Use of dithiolylidene malonate derivatives for the prevention of metastasis.**

(57) A pharmaceutical for preventing metastasis of cancer which contains as active ingredient a dithiol derivative represented by the general formula (I):

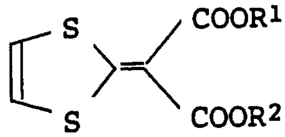

(I)

wherein $R^1$ and $R^2$, which may be the same or different, are alkyl groups having 1 to 6 carbon atoms; and a method for preventing metastasis of cancer by the use of said pharmaceutical.

EP 0 585 728 A1

## BACKGROUND OF THE INVENTION

### Field of the Invention

The present invention relates to a pharmaceutical for preventing metastasis of cancer which contains as active ingredient a dithiol derivative represented by the general formula (I):

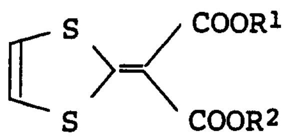

(I)

wherein $R^1$ and $R^2$, which may be the same or different, are alkyl groups having 1 to 6 carbon atoms; and a method for preventing metastasis of cancer by the use of said pharmaceutical.

### Related Art Statement

The compound used in the present is known to have bactericidal activity for agricultural and horticultural use in Japanese Patent Examined Publication No. 54-43506, curing activity on liver disease in Japanese Patent Examined Publication US 4,118,506, and anticancer activity in Japanese Patent Examined Publication No. 63-66287. However, the inhibitory activity on metastasis of cancer described in the present specification is not known in itself.

## SUMMARY OF THE INVENTION

Surgical treatment, radiotherapy, etc. for primary lesions of cancer have been greatly advanced, but there is no effective means for preventing metastasis of cancer.

Metastasis of cancer is caused via liberation of cancerous cells from a primary cancer, their migration through vessels, attachment to organs, in filtration, multiplication, etc.

The present inventors earnestly investigated a substance capable of preventing the infiltration of cancerous cells, as one of agents for preventing metastasis of cancer, and consequently found that dithiol derivatives of the general formula (I) have inhibitory effect on metastasis of cancer, whereby the present invention has been accomplished.

## DETAILED EXPLANATION OF THE INVENTION

The present invention relates to a use of a dithiol derivative of the above general formula (I) as a pharmaceutical for preventing metastasis of cancer, a pharmaceutical for preventing metastasis of cancer which contains said derivative as active ingredient, and a method for preventing metastasis of cancer by the use of said pharmaceutical.

Organs to which cancer tends to metastasize include lungs, liver, digestive organs, etc. The compound used in the present invention has an excellent inhibitory effect on metastasis of cancer to these organs.

As model systems for measuring the degree of metastasis of cancer, there have been made up experimental systems for metastasis of cancer using B16 melanoma, Lewis lung cancerous cells, rat mammary cancerous cells SST-2, etc. [Cancer and Chemotherapy, 12(6), 1210-1221, 1985]. The present inventors carried out screening by using rat mammary cancerous cells SST-2 in a model system for spontaneous metastasis of cancer developed by Kobayashi et al., and has accomplished the present invention.

In the compound of the above general formula (I), the alkyl group having 1 to 6 carbon atoms includes methyl group, ethyl group, i-propyl group, n-propyl group, n-butyl group, i-butyl group, s-butyl group, n-pentyl group, n-hexyl group, etc.

Typical examples of the compound of the above general formula (I) are as follows:

General formula (I)

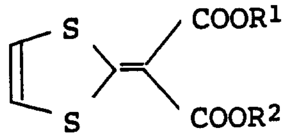

(I)

| Compound No. | $R^1$ | $R^2$ | Melting point or refractive index |
|---|---|---|---|
| 1 | $CH_3$ | $CH_3$ | m.p. 134.5-135 °C |
| 2 | $CH_3$ | i-$C_4H_9$ | $n_D^{20}$ : 1.5928 |
| 3 | $C_2H_5$ | $C_2H_5$ | m.p. 113 °C |
| 4 | $C_2H_5$ | i-$C_3H_7$ | m.p. 57-58 °C |
| 5 | i-$C_3H_7$ | i-$C_3H_7$ | m.p. 59-60 °C |
| 6 | i-$C_4H_9$ | i-$C_4H_9$ | m.p. 76-78 °C |
| 7 | i-$C_5H_{11}$ | i-$C_5H_{11}$ | m.p. 55-56 °C |
| 8 | n-$C_3H_7$ | n-$C_3H_7$ | m.p. 73-75 °C |
| 9 | n-$C_4H_9$ | n-$C_4H_9$ | m.p. 74-75 °C |
| 10 | s-$C_4H_9$ | s-$C_4H_9$ | m.p. 68-65 °C |
| 11 | n-$C_5H_{11}$ | n-$C_5H_{11}$ | m.p. 70-70.5 °C |

The compound of the above general formula (I) has only low toxity to warm-blooded animals. For example, its acute toxity (LD50) in mice (♂) in the case of oral administration ranges from 1,000 to 6,000 mg/kg or is below this range.

Said compound has no undesirable influence on a test warm-blooded animal in a usual dose range. The pharmaceutical for preventing metastasis of cancer of the present invention has an excellent inhibitory effect on metastasis of cancer in a warm-blooded animal and hence a marked life-prolonging effect on the warm-blooded animal suffering from the cancer.

The pharmaceutical for preventing metastasis of cancer of the present invention may be composed of a compound of the general formula (I) alone or a mixture of said compound, pharmaceutically acceptable inactive carriers or diluents, and/or other pharmacologically active ingredients. The pharmaceutical may be prepared in dosage unit form. As forms which the pharmaceutical may have as a medicinal composition, there can be exemplified powder, granules, tablets, dragees, capsules, pills, suspensions, solutions, emulsions, ampules, injections and isotonic solutions.

The diluents used for preparing the pharmaceutical for preventing metastasis of cancer of the present invention are pharmaceutically acceptable and are materials other than the compound according to the present invention. They may be any of solids, semisolids, liquids or ingestible capsules and includes various materials, for example, excipients, extenders, binders, wetting agents, disintegrators, surfactants, lubricants, dispersants, buffers, taste-improvers, odor-improvers, coloring matters, perfumes, preservatives, dissolution assistants, solvents, coatings, frostings, etc. But the diluents are not limited thereto. These materials can be used singly or as a mixture thereof.

The pharmaceutical for preventing metastasis of cancer which is obtainable according to the present invention may be prepared by any known method. For example, the active ingredient is mixed with the diluents to yield, for instance, granules, and the composition thus obtained is formed, for example, into tablets. When the pharmaceutical is used as a parenteral drug, it should be sterilized. If necessary, it should be isotonic with regard to blood.

The parenteral administration includes injection (including, for example, subcutaneous injection, intramuscular injection, intravenous injection and drip) and per anum administration (suppositories).

Since the compound used in the present invention is applicable in itself as a pharmaceutical for preventing metastasis of cancer, the active ingredient is contained in the composition according to the present invention usually in an amount of 0.01 to 100% by weight.

The dose of the pharmaceutical for preventing metastasis of cancer of the present invention is varied depending on age, sex, body weight, administration route, etc. In the case of oral administration and administration of a suppository, the pharmaceutical may be administered to an adult usually in a dose of 0.25 to 150 mg, preferably 1 to 50 mg per kg of body weight per day in one portion or several portions. In the case of intravenous injection and dip, the pharmaceutical may be administered to an adult usually in a

dose of 0.025 to 10 mg, preferably 0.25 to 10 mg per kg of body weight per day in one portion or several portions.

Thus, the method for preventing metastasis of cancer can be established by administering an effective amount of the pharmaceutical containing a dithiol derivative of the general formula (I) as active ingredient, in consideration of its use, the condition of a patient, etc., for example, orally, percutaneously, or by intravenous injection.

Formulation examples are described below but they are not intended in any way to limit the scope of the invention.

Formulation Example 1

An injection is obtained by dissolving 10 g of a compound according to the present invention which has been sterilized, in olive oil for injection under sterile conditions to obtain a 100 ml of a solution, and filling 10-ml ampules for injection with the solution.

Formulation Example 2

Drops are obtained by dissolving 0.5 g of a compound according to the present invention which has been sterilized and 0.6 g of an ethylene oxide adduct of hardened castor oil in 500 ml of a 5% glucose solution for drip, and sterilizing the resulting solution by filtration.

Formulation Example 3

Capsules are obtained by mixing 200 mg of a compound according to the present invention, 70 mg of corn starch and 5 mg of magnesium stearate, and packing the resulting mixture into gelatin capsules.

Formulation Example 4

A powder is obtained by mixing 10 g of a compound according to the present invention with 10 g of crystalline lactose.

Formulation Example 5

Film-coated tablets are obtained by mixing 200 mg of a compound according to the present invention, 20 mg of potato starch, 20 mg of crystalline cellulose, 20 mg of lactose, 15 mg of anhydrous calcium hydrogen phosphate, 5 mg of sucrose fatty acid ester and 3 mg of magnesium aluminate metasilicate, tableting the resulting mixture, and coating the tablets with 10 mg of hydroxypropylmethyl cellulose.

Formulation Example 6

An oral syrup is obtained by suspending a compound according to the present invention in syrup for oral administration.

The effect of the method for preventing metastasis of cancer of the present invention is explained below with test examples.

Test Example 1

Rat lung blood vessel endothelial cells (RLE) were pretreated with compound 5 according to the present invention in an amount of 1, 10 or 20 ng per ml of RLE for 24 hours, washed, and then inoculated (treated) with rat spontaneous mammary cancer cells (SST-2), followed by cultivation. Thereafter, the degree of infiltration of SST-2 into RLE was investigated by counting colonies, whereby the ability (activity) of the compound according to the present invention to inhibit the infiltrating ability of SST-2 was checked. The results obtained are shown in Table 1.

Table 1

| Dose of a compound according to the invention (ng/ml) | Number of colonies/$cm^2$ (average ± standard deviation) |
|---|---|
| 0 | 167.5 ± 28.9 |
| 1 | 178.3 ± 12.6 |
| 10 | 48.8 ± 10.4 |
| 20 | 38.3 ± 7.6 |

The compound according to the present invention significantly prevented the infiltration of SST-2 into RLE at a dose of 10 ng/ml or more. Therefore, it is clear that the compound prevents the cancerous cells from metastasizing to other organs.

Test Example 2

Rats suffering from spontaneous hypertension (SHR rats) (groups of 5 rats each) were implanted subcutaneously on the back with rat spontaneous mammary cancer cells (SST-2) at a rate of 1 x $10^5$ cells/rat. After 35 days, the rats were killed and the weight of the lungs and the number of colonies formed were examined, whereby the degree of metastasis of SST-2 to the lungs was investigated. Compound 5 according to the present invention was orally administered to the groups to be treated with compound 5, in a dose of 30 or 60 mg/rat from 7 days before the implantation to the 34th day after the implantation, and the weight of subcutaneous tumor, the weight of the lungs, and the number of colonies formed by metastasis were compared with those in an untreated control group. The results obtained are shown in Table 2.

Table 2

| Dose of a compound according to the invention mg/rat | Indication of metastasis | | |
|---|---|---|---|
| | Weight of subcutaneous tumor (g ± standard deviation) | Weight of lungs (g ± standard deviation) | Number of tumor colonies formed in lungs (average ± standard deviation) |
| 0 | 32.6±9.6 | 3.6±1.5 | Too many to be counted |
| 30 | 50.5±10.1 | 1.5±0.1 | 1.3±1.5 |
| 60 | 38.5±15.7 | 1.4±0.1 | 2.4±2.4 |

In the untreated control group, the weight of the lungs was increased by the metastasis of SST-2, and the number of colonies formed was too large to be counted. On the other hand, in the groups treated with the compound according to the present invention, the multiplication of subcutaneous tumor was not suppressed, but the weight of lung tumor was significantly lighter than in the untreated control group and the number of tumor colonies formed was significantly smaller than in the untreated control group. From these results, it is clear that the compound according to the present invention prevents the metastasis to the lungs without suppressing the multiplication of cancerous cells of the primary cancer.

Test Example 3

SHR rats (groups of 5 rats each) were implanted intravenously with SST-2 at a rate of 5 x $10^4$ cells/rat. After 20 days, the rats were killed and the weight of the lungs and the number of colonies formed were examined. Compound 5 according to the present invention was orally administered to the groups to be treated with compound 5, in a dose of 60 mg/rat from the 1st day to the 19th day after the implantation, and the weight of the lungs and the number of colonies formed by metastasis were compared with those in an untreated control group. The results obtained are shown in Table 3.

Table 3

| Dose of a compound according to the invention mg/rat | Indication of metastasis | |
|---|---|---|
| | Weight of lungs (g ± standard deviation) | Number of tumor colonies formed in lungs (average ± standard deviation) |
| 0<br>60 | 1.1±0.1<br>1.2±0.3 | 89.8±86.8<br>39.2±21.1 |

In both the group treated with the compound according to the present invention and the untreated control group, there was no significant change in the weight of the lungs. But, in the group treated with the compound according to the present invention, the number of colonies formed was significantly smaller than in the untreated control group. Therefore, it is clear that said compound prevents the metastasis to the lungs.

Test Example 4

SST-2 were implanted in the spleens of SHR rats (groups of 5 rats each) at a rate of 1 x $10^5$ cells/rat. After 20 days, the rats were killed, and the weight of intraperitoneal tumor and the number of tumor colonies formed in the liver were examined and the existence of ascites was investigated. Compound 5 according to the present invention was orally administered to the groups to be treated with compound 5, in a dose of 60 mg/rat from the 1st day to the 19th day after the implantation, and the weight of intraperitoneal tumor, the number of tumor colonies formed in the liver, and the existence of blood-like ascites were compared with those in an untreated control group. The results obtained are shown in Table 4.

Table 4

| Dose of a compound according to the invention mg/rat | Indication of metastasis | | |
|---|---|---|---|
| | Weight of intraperitoneal tumor (g ± standard deviation) | Number of tumor colonies formed in liver (average ± standard deviation) | Blood-like ascites |
| 0<br>60 | 7.1±5.9<br>1.9±1.7 | 84.0±18.8<br>48.2±41.8 | +<br>- |

In the group treated with the compound according to the present invention, the weight of intraperitoneal tumor was lighter than in the untreated control group and the number of tumor colonies formed was smaller than in the untreated control group. In addition, no ascites was observed. From these results, it is clear that the compound according to the present invention prevents metastasis to the liver by the implantation in the spleen.

Dithiol derivatives of the general formula (I) according to the present invention are useful as agents for preventing metastasis of cancer.

## Claims

1. A method for preventing metastasis of cancer by applying an effective amount of a dithiol derivative represented by the general formula (I):

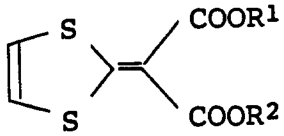

(I)

wherein $R^1$ and $R^2$, which may be the same or different, are alkyl groups having 1 to 6 carbon atoms.

**2.** A pharmaceutical for preventing metastasis of cancer which comprises as active ingredient a dithiol derivative represented by the general formula (I):

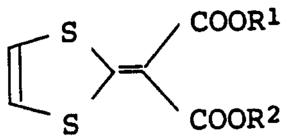

(I)

wherein $R^1$ and $R^2$, which may be the same or different, are alkyl groups having 1 to 6 carbon atoms.

**3.** A use of a dithiol derivative represented by the general formula (I):

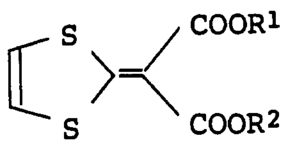

(I)

(wherein $R^1$ and $R^2$, which may be the same or different, are alkyl groups having 1 to 6 carbon atoms) for preparing a pharmaceutical for preventing metastasis of cancer.

European Patent Office

# EUROPEAN SEARCH REPORT

Application Number
EP 93 11 3176

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | PATENT ABSTRACTS OF JAPAN (C-112)(6122) 7 July 1982 & JP-A-57 050 916 (NIPPON NOHYAKU CO LTD) 25 March 1982 * abstract * --- | 1-3 | A61K31/385 |
| D,X | US-A-4 118 506 (K. TANINAKA ET AL.) 3 October 1978 * the whole document * --- | 2 | |
| X | J. GASTROENTEROL. HEPATOL. vol. 1, no. 5 , 1986 pages 379 - 383 T. TAKINO ET AL 'Effect of diisopropyl 1,3-dithiol-2-ylidenemalonate on rat liver ans AFP-positive cells in 3'-Me-DAB hepatocarcinogenesis.' * the whole document * ----- | 1-3 | |
| | | | TECHNICAL FIELDS SEARCHED (Int.Cl.5) |
| | | | A61K |

The present search report has been drawn up for all claims

| Place of search | Date of completion of the search | Examiner |
|---|---|---|
| THE HAGUE | 8 November 1993 | KLAVER, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)